(19) Europäisches Patentamt
European Patent Office
Office européen des brevets

(11) **EP 1 983 340 A1**

(12) **EUROPÄISCHE PATENTANMELDUNG**

(43) Veröffentlichungstag:
**22.10.2008 Patentblatt 2008/43**

(51) Int Cl.:
**G01N 33/18** *(2006.01)*    **G01N 35/00** *(2006.01)*
**G01N 31/22** *(2006.01)*

(21) Anmeldenummer: **07106535.3**

(22) Anmeldetag: **19.04.2007**

(84) Benannte Vertragsstaaten:
**AT BE BG CH CY CZ DE DK EE ES FI FR GB GR HU IE IS IT LI LT LU LV MC MT NL PL PT RO SE SI SK TR**
Benannte Erstreckungsstaaten:
**AL BA HR MK RS**

(71) Anmelder: **Hach Lange GmbH**
**40549 Düsseldorf (DE)**

(72) Erfinder:
 • **Huenig, Isabel**
 **01159, Dresden (DE)**

 • **Frömel, Rainer**
 **53842, Troisdorf (DE)**
 • **Farjam, Aria**
 **40223, Düsseldorf (DE)**

(74) Vertreter: **Fitzner, Uwe et al**
**Dres. Fitzner & Münch**
**Patent- und Rechtsanwälte**
**Hauser Ring 10**
**40878 Ratingen (DE)**

(54) **Verfahren zur Bestimmung der Konzentration von Analyten**

(57)    Die vorliegende Erfindung betrifft ein Verfahren zur Bestimmung der Konzentration von Analyten, wobei
- der Analyt mit einem Indikator in Kontakt gebracht wird,
- der Indikator in Form eines Feststoffgemisches vorgelegt wird, das den Indikator und magnetische Partikel enthält und weitere Substanzen enthalten kann,
- ein Binden und/oder Anreichern des Indikators, der mit dem Analyten reagiert hat, auf den magnetischen Partikeln stattfindet,
- solche magnetischen Partikel eingesetzt werden, die eine Erkennbarkeit oder Messbarkeit der Indikatoränderung ermöglichen,
- nach der Reaktion des Indikators mit dem Analyten und des Binden und/oder Anreichern des Indikators auf den magnetischen Partikeln mittels eines Magnetfeldes eine Aufkonzentrierung (Separierung) der Partikel aus der Lösung erfolgt und
- die Veränderung der magnetischen Partikel mittels optischer Methoden gemessen wird.

*Fig. 2*

**Beschreibung**

Die vorliegende Erfindung betrifft ein Verfahren zur Bestimmung der Konzentration von Analyten, insbesondere in Wasser mittels optischer Messverfahren sowie magnetische Partikel zur Durchführung desselben.

Aus dem Stand der Technik sind verschiedene Verfahren zur Bestimmung der Konzentration von Analyten in Wasser bekannt. U. a. werden photometrische Testkits (Küvetten-Tests) und Teststreifen verwendet. Diesbezüglich sei beispielsweise auf die DE 199 06 151 A1, DE 196 16 760 A1, DE 43 078 14 A1, DE 100 18 784 C2, DE 10 12199 A1, EP 066 3239 B1 sowie WO 00/75653 verwiesen.

Küvettentests mit photometrischer Auswertung zeichnen sich dadurch aus, dass sie ein sehr verlässliches, digitales Ergebnis liefern, während die Vorteile der Teststreifen darin liegen, dass sie kostengünstig produziert werden können, einfach zu handhaben sowie zu entsorgen sind. Es ist erstrebenswert, die Vorteile beider Methoden in einem System zu verwirklichen. Demgemäß wird erfindungsgemäß eine Miniaturisierung des Küvettentests auf Teststreifengröße mit automatisierter Auswertung angestrebt.

Solche Systeme nutzen sogenannte "Indikatoren" zur Bestimmung der Analytkonzentration. Die Indikatoren erfahren aufgrund der Anwesenheit von Analytmolekülen eine optische Veränderung. Bei der optischen Veränderung des Indikators handelt es sich in der Regel um eine Veränderung der Lichtabsorption, der Fluoreszenz oder der Lumineszenz.

Gegenstand der Erfindung ist ein Verfahren zur Bestimmung der Konzentration von Analyten, wobei

- der Analyt mit einem Indikator in Kontakt gebracht wird,
- der Indikator in Form eines Feststoffgemisches vorgelegt wird, das den Indikator und magnetische Partikel enthält und weitere Substanzen enthalten kann,
- ein Binden und/oder Anreichern des Indikators, der mit dem Analyten reagiert hat, auf den magnetischen Partikeln stattfindet,
- solche magnetischen Partikel eingesetzt werden, die eine Erkennbarkeit oder Messbarkeit der Indikatoränderung ermöglichen,
- nach der Reaktion des Indikators mit dem Analyten und des Binden und/oder Anreichern des Indikators auf den magnetischen Partikeln mittels eines Magnetfeldes eine Aufkonzentrierung (Separierung) der Partikel aus der Lösung erfolgt und
- die Veränderung der magnetischen Partikel mittels optischer Methoden gemessen wird.

Bei dem erfindungsgemäßen Verfahren werden Feststoffgemische (Reagenzien) vorgelegt, welche magnetische Partikel und Farbindikatoren enthalten, aber auch noch weitere Substanzen enthalten können, wie zum Beispiel Puffersubstanzen, Maskierungsmittel oder Trägersubstanzen. Die Ummantelung der magnetischen Partikel ist vorteilhafterweise so gestaltet, dass sie genug hydrophile Bereiche hat, so dass sie im Wasser suspendierbar sind. Zusätzlich muss sie Bereiche haben, zu denen der Farbindikator eine höhere Affinität hat, als zu Suspensionsmedien, z B. zu Wasser. Dies kann z. B. durch kationische, anionische oder hydrophobe Wechselwirkungen oder durch molekulare Erkennung gewährleistet sein.

Vorteilhaft ist es, wenn die Partikel superparamagnetisch sind, da sie dann nicht durch eigene Magnetisierung aneinander haften bleiben.

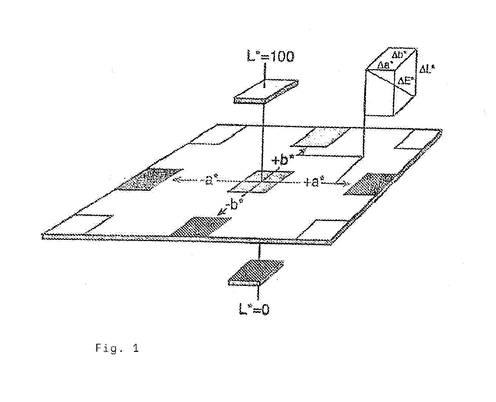

Erfindungsgemäß müssen die magnetischen Partikel derart ausgestaltet sein, dass mittels üblicher Messmethoden die Änderungen des auf ihnen angereicherten Indikators erkennbar sind. Insbesondere sollten Farbänderungen des Indikators mittels optischer Methoden bestimmbar sein. Zu diesem Zweck sollten magnetische Partikel eingesetzt werden, die eine möglichst helle Farbschattierung aufweisen. Durch den Einsatz vorzugsweise weißer, grauer, hell gefärbter oder durchsichtiger Partikel wird die Verwendung von aus dem Stand der Technik bekannten Farbindikatoren ermöglicht. Besonders bevorzugt sind weiße oder durchsichtige magnetische Partikel. Geeignet sind jedoch auch die verschiedenen Grauabstufungen zwischen schwarz und weiß. Neben den zwischen grau und schwarz liegenden Grauabstufungen sind auch vorzugsweise helle Farben geeignet, z. B. gelb oder auch grün. Die farbliche Eignung der magnetischen Partikel lässt sich beispielsweise anhand der CIELAB-Farbabstandsformel bestimmen (vgl. RÖMPP - Lacke und Druckfarben 1998, Seite 114, vgl. auch Erläuterungen zur Fig 1). Erfindungsgemäß sind Partikel, welche schwarz sind, in der Regel kaum geeignet.

Die durchschnittliche Größe der magnetischen Partikel liegt zwischen 0,1 und 100 $\mu$m, vorzugsweise zwischen 0,7 $\mu$m und 5 $\mu$m. Vorzugsweise sind die Partikel so gestaltet, dass sie in Wasser nur langsam sedimentieren und beim Durchmischen in Suspension gehalten werden können. Gleichzeitig sind die Partikel schwer genug, um innerhalb kurzer Zeit magnetisch aufkonzentriert (separiert) werden zu können. Vorzugsweise liegt das Reagenz mit den magnetischen Partikeln in trockener Form, z. B. als Lyophilisat vor.

Die magnetischen Partikel lassen sich ohne hohen Energie- oder mechanischen Aufwand aufkonzentrieren. Zur Aufkonzentrierung der magnetischen Partikel wird erfindungsgemäß nach der Reaktion des Indikators mit dem Analyten und der Bindung und/oder Anreicherung des Indikators auf den magnetischen Partikeln ein Magnetfeld eingesetzt. Dessen Flussdichte liegt zwischen 10 und 500mT, vorzugsweise zwischen 35 und 45mT.

Als Indikatoren können die aus dem Stand der Technik bekannten Stoffe verwendet werden. Besonders bevorzugt sind Indikatoren, die in der Photometrie verwendet werden (s. z.B. Z. Marczenko, Separation and Spectrophotometric Determination of Elements, 1986, p. 1-678, wie zum Beispiel N,N- Diethyl-1,4-Benzenediamine (DPD) und Tetramethylbenzidine zur quantitativen Bestimmung von Chlor in Wasser, 2,6-Dimethylphenol für die Bestimmung von Nitrat, Ammoniummolybdat für die Bestimmung von Phosphat oder Salicylsäure/Nitroprussidnatrium für die Bestimmung von Ammonium).

Die Farbänderung der Indikatoren kann mit üblichen Methoden gemessen werden. Erfindungsgemäß handelt es sich hierbei um optische Methoden. Beispiele hierfür sind Photometrie, Reflektometrie, Flourimetrie, Luminometrie und Colorimetrie.

Gegenstand der Erfindung ist schließlich auch die Verwendung von geeigneten magnetischen Partikeln für die Bestimmung der Konzentration von Analyten, insbesondere bei der Analyse von Wasserproben. Diese Partikel sind in der zuvor beschriebenen Form ausgestaltet.

Im Folgenden wird die Erfindung unter Bezugnahme auf die Figuren beispielhaft näher erläutert:

Fig. 1 zeigt die Konstruktion des Farbabstands im CIELAB-System

Fig. 2 zeigt die Aufsicht auf einen Chip, der zur Konzentraionsmessung von Analyten verwendet werden kann.

Das System zur Fig. 1 bietet ein Verfahren zur quantitativen Bestimmung von Farbabständen bei Körperfarben. Der Farbabstand $\Delta E_{ab}$ nach DIN 6174: 1979 - 01 im CIELAB-System wird als Raumdiagonale berechnet. Die an der genannten Literaturstelle angegebene Formel lautet:

$$\Delta E_{ab} = [(\Delta L^*)^2 + (\Delta a^*)^2 + (\Delta b^*)^2]^{1/2}.$$

Der Farbenraum wird durch L\*, a\*, b\* bestimmt. L\*=0 bedeutet schwarz. L\*=100 ist weiß. Dazwischen liegen die verschiedenen Grauabstufungen. -b\*sind die Abstufen zu blau, +b\* zu gelb, -a\* zu grün und +a\* zu rot. Erfindungsgemäß kommen daher die Bereiche L\*>0 bis L\*=100 sowie +b\* und -a\* in Betracht. Höchst bevorzugt sind L\*=100 sowie durchsichtige Varianten.

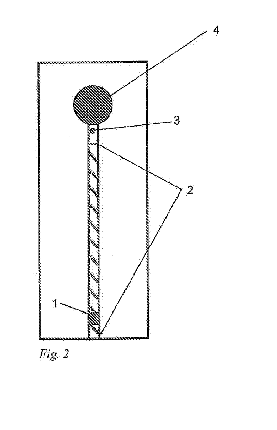

Zur Quantifizierung der Konzentration eines Analyten werden 10 $\mu$l einer Probe in eine mikrofluidische Kanalstruktur aus Kunststoff eingesaugt. Diese ist schematisch in Fig. 2 dargestellt. Es handelt sich um die Aufsicht auf einen Chip, der zur Konzentrationsbestimmung von Analyten verwendet werden kann. Das Reagenz, welches magnetische Partikeln und Indikator enthält wird in fester Form an Position 1 vorgelegt. In dem Bereich 2 erfolgt die Durchmischung mit der Probe. Mittels des Magneten 3 erfolgt die Aufkonzentrierung der Partikel auf einen Messfleck. Die Kanalstruktur besteht aus einem 20 mm langen und 1x1 mm² großen Kanal, der an einem Anschluss für eine Pumpe endet. Durch Betätigen einer Pumpe wird die Probe in und durch den Kanal bewegt.

Ein Reagenz mit 0,064 $\mu$l weißen, magnetischen Partikeln 1 liegt in fester Form im Bereich nahe der Kanalöffnung vor und wird durch Hin- und Herpumpen der Probenflüssigkeit innerhalb der 18 $\mu$l Kanalstruktur, die dem Eingang des Kanals zugewandt ist gelöst und suspendiert. Die Partikel haben einen Durchmesser von ca. 1 $\mu$m.

Während der Durchmischung reagiert der Farbindikator mit dem Analyten zu einer Substanz, die anders gefärbt ist als die Ausgangsmaterialien Gleichzeitig wird das Reaktionsprodukt aus Indikator und Analyt an den weißen magnetischen Partikeln adsorbiert. Anschließend wird die Suspension über ein 0,4 x 0,4 mm² großes Magnetfeld bewegt, das an der Unterseite des Chips außerhalb des Kanals angelegt wird. Dabei werden die magnetischen Partikel an dem Magnetfeld auf einem Messfleck von 0,4 x 0,4 mm² abgeschieden.

Durch die Aufkonzentrierung des Indikators von einem Probevolumen von 10 $\mu$l auf ein Partikelvolumen von 0,064 $\mu$l sind diese deutlich intensiver gefärbt als die Lösung. Die Farbintensität wird colorimetrisch in Reflektion gemessen.

**Patentansprüche**

1. Verfahren zur Konzentrationsbestimmung von Analyten, wobei

   - der Analyt mit einem Indikator in Kontakt gebracht wird,
   - der Indikator in Form eines Feststoffgemisches vorgelegt wird, das den Indikator und magnetische Partikel enthält und weitere Substanzen enthalten kann,
   - eine Bindung und/oder Anreicherung des Indikators, der mit dem Analyten reagiert hat, auf den magnetischen Partikeln stattfindet,
   - solche magnetischen Partikel eingesetzt werden, die eine Erkennbarkeit oder Messbarkeit der Indikatoränderung ermöglichen,
   - nach der Reaktion des Indikators mit dem Analyten und der Bindung und/oder Anreicherung des Indikators auf den magnetischen Partikeln mittels eines Magnetfeldes eine Aufkonzentrierung (Separierung) der Partikel aus der Lösung erfolgt und
   - die Veränderung der magnetischen Partikel mittels optischer Methoden gemessen wird.

2. Verfahren nach Anspruch 1 **dadurch gekennzeichnet,**
   **dass** der Indikator auf die Oberfläche der magnetischen Partikel aufgebracht ist.

**3.** Verfahren nach einem der vorhergehenden Ansprüche **dadurch gekennzeichnet,** **dass** Partikel eingesetzt werden, deren Oberfläche Bereiche aufweist, zu denen der Farbindikator eine höhere Affinität als zum Suspensionsmedium hat.

**4.** Verfahren nach einem der vorhergehenden Ansprüche **dadurch gekennzeichnet,** **dass** Indikatoren verwendet werden, deren Farbänderung messbar ist.

**5.** Verfahren nach einem der vorhergehenden Ansprüche **dadurch gekennzeichnet,** **dass** magnetische Partikel eingesetzt werden, welche einen Farbton aufweisen, welche nach dem CIELAB-System einen Wert von $L^* > 0$ aufweisen.

**6.** Verfahren nach einem der vorhergehenden Ansprüche **dadurch gekennzeichnet,** **dass** die magnetischen Partikel eingesetzt werden, die weiß oder durchsichtig sind.

**7.** Verfahren nach einem der vorhergehenden Ansprüche **dadurch gekennzeichnet,** **dass** die magnetischen Partikel eine Größe von 0,1 bis 100 $\mu$m haben.

**8.** Verfahren nach einem der vorhergehenden Ansprüche **dadurch gekennzeichnet,** **dass** eine magnetische Flussdichte von 10 bis 500mT zur magnetischen Filtration verwendet wird.

**9.** Verfahren nach einem der vorhergehenden Ansprüche **dadurch gekennzeichnet,** **dass** die Indikatoränderung photometisch, colorimetrisch, luminometrisch, flourimetrisch oder reflektrometrisch gemessen wird.

**10.** Magnetische, suspendierbare Partikel zur Durchführung des Verfahrens nach einem der vorhergehenden Ansprüche, wobei
die Partikel derart ausgestaltet sind, dass eine Erkennbarkeit oder Messbarkeit von Indikatoränderungen ermöglicht ist, und dass deren Oberfläche eine höhere Affinität zum Farbindikator als zum Suspensionsmedium aufweist.

**11.** Partikel nach Anspruch 10 **dadurch gekennzeichnet,** **dass** sie einen Indikator enthalten oder mit einem solchen beschichtet sind.

**12.** Partikel nach Anspruch 10 **dadurch gekennzeichnet,** **dass** deren Oberfläche hydrophile Bereiche aufweist.

**13.** Partikel nach einem der vorhergehenden Ansprüche 10 bis 12 **dadurch gekennzeichnet,** **dass** sie in trockener, fester Form vorliegen.

**14.** Partikel nach einem der vorhergehenden Ansprüche 10 bis 13 **dadurch gekennzeichnet,** **dass** Indikator und magnetische Partikel in Form eines Lyophilisats vorliegen.

**15.** Partikel nach Anspruch 10 **dadurch gekennzeichnet,** **dass** sie nach der CIELAB-Farbabstandsformel einen Wert von $L^* > 0$ aufweisen oder weiß oder durchsichtig sind.

**16.** Partikel nach einem der Ansprüche 10 bis 15 **dadurch gekennzeichnet,** **dass** sie eine Größe von 0,1 bis 100 $\mu$m aufweisen.

**17.** Verwendung der Partikel nach einem der Ansprüche 10 bis 16 zur Bestimmung der Konzentration von Analyten, vorzugsweise in Wasser.

Fig. 1

Fig. 2

**Europäisches Patentamt**

# EUROPÄISCHER RECHERCHENBERICHT

Nummer der Anmeldung

EP 07 10 6535

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | EP 0 893 692 A1 (STRATEC ELEKTRONIK GMBH [DE] STRATEC BIOMEDICAL SYSTEMS AG [DE]) 27. Januar 1999 (1999-01-27) * Zusammenfassung * * Spalte 1, Zeilen 12-32 * * Spalte 4, Zeile 53 - Spalte 6, Zeile 1 * | 1,4-7,9, 10,13, 15-17 | INV. G01N33/18 G01N35/00 ADD. G01N31/22 |
| Y | ----- | 2,3,11 | |
| Y | US 4 731 337 A (LUOTOLA JUHANI E I [FI] ET AL) 15. März 1988 (1988-03-15) * Zusammenfassung * * Spalte 1, Zeilen 5-9,31-34 * | 2,3,11 | |
| X | ----- US 2003/077598 A1 (PHAN BRIGITTE CHAU [US] ET AL) 24. April 2003 (2003-04-24) * Zusammenfassung * * Absätze [0010], [0025], [0027] * * Absatz [0045]; Abbildung 20C * | 1,10 | |
| X | * Absatz [0148] * | 14 | |
| X | * Absatz [0015] * | 17 | |
| X | ----- US 2005/191687 A1 (WANG TIANXIN [US] ET AL) 1. September 2005 (2005-09-01) * Zusammenfassung; Abbildung 6 * * Absätze [0006], [0063] * | 1,10, 1712 | G01N |
| X | * Absatz [0040] * | 12 | |
| X | ----- DE 10 2004 044048 A1 (QUALIS LAB GMBH [DE]) 30. März 2006 (2006-03-30) * Zusammenfassung; Abbildungen A,B,C * * Absatz [0002]; Anspruch 1 * | 1,10,17 | |
| A | ----- US 6 254 830 B1 (PIVARNIK PHILIP [US] ET AL) 3. Juli 2001 (2001-07-03) * Zusammenfassung * * Spalte 2, Zeilen 54-64 * * Spalte 6, Zeilen 31-34,45-47 * ----- | 1-17 | |

RECHERCHIERTE SACHGEBIETE (IPC)

Der vorliegende Recherchenbericht wurde für alle Patentansprüche erstellt

| Recherchenort | Abschlußdatum der Recherche | Prüfer |
|---|---|---|
| München | 2. Oktober 2007 | Hanisch, Christian |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**

EP 07 10 6535

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

02-10-2007

| Im Recherchenbericht angeführtes Patentdokument | | Datum der Veröffentlichung | Mitglied(er) der Patentfamilie | | Datum der Veröffentlichung |
|---|---|---|---|---|---|
| EP 0893692 | A1 | 27-01-1999 | KEINE | | |
| US 4731337 | A | 15-03-1988 | DE | 3584196 D1 | 31-10-1991 |
| | | | EP | 0169434 A2 | 29-01-1986 |
| | | | JP | 2584611 B2 | 26-02-1997 |
| | | | JP | 61041966 A | 28-02-1986 |
| US 2003077598 | A1 | 24-04-2003 | US | 2005266476 A1 | 01-12-2005 |
| US 2005191687 | A1 | 01-09-2005 | KEINE | | |
| DE 102004044048 | A1 | 30-03-2006 | KEINE | | |
| US 6254830 | B1 | 03-07-2001 | US | 6630355 B1 | 07-10-2003 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19906151 A1
- DE 19616760 A1
- DE 4307814 A1
- DE 10018784 C2
- DE 1012199 A1
- EP 0663239 B1
- WO 0075653 A

**In der Beschreibung aufgeführte Nicht-Patentliteratur**

- *vgl. RÖMPP - Lacke und Druckfarben,* 1998, 114
- **B. Z. MARCZENKO.** *Separation and Spectrophotometric Determination of Elements,* 1986, 1-678